# EUROPÄISCHE PATENTSCHRIFT

(11) **EP 0 675 205 B1**
(45) Veröffentlichungstag und Bekanntmachung des Hinweises auf die Patenterteilung: **01.10.1997**
(21) Anmeldenummer: 95104165.6
(22) Anmeldetag: 22.03.1995
(51) Int. Cl.: C12P 41/00, C12P 7/62, C12P 7/02

(54) **Enzymatische Racematspaltung asymmetrischer Alkohole mittels Vinylestern mehrbasiger Carbonsäuren**
Enzymatic separation of racemates of asymmetric alcohols using vinyl esters of poylcarboxylic acids
Dédoublement enzymatique de racémates d'alcools asymétriques au moyen d'esters vinyliques d'acides polycarboxyliques

(30) Priorität: 30.03.1994 AT 672/94
(43) Veröffentlichungstag der Anmeldung: 04.10.1995
(73) Patentinhaber: DSM Chemie Linz GmbH, 4021 Linz (AT)
(72) Erfinder: Seufer-Wasserthal, Peter, Dipl.-Ing. Dr., A-4690 Schwanenstadt (AT); Mayrhofer, Herbert, A-4210 Engerwitzdorf (AT); Wirth, Irma, A-4470 Enns (AT); Pöchlauer, Peter, Dr., A-4020 Linz (AT)
(74) Vertreter: Kunz, Ekkehard, Dr.

(56) Entgegenhaltungen:
- EP-A- 0 118 163
- EP-A- 0 321 918
- EP-A- 0 577 446

## Beschreibung

Asymmetrische Alkohole sind wichtige Zwischenverbindungen für eine Reihe pharmazeutisch wirksamer Verbindungen, beispielsweise für Substanzen, die das zentrale Nervensystem (ZNS) beeinflussen. Die Erkenntnis, daß üblicherweise jeweils nur eine enantiomere Form dieser Substanzen die erwünschte pharmazeutische Wirkung ausübt, hat zu verstärkter Nachfrage nach Herstellverfahren ihres aktiven Enantiomers unter Vermeidung der Bildung ihres inaktiven, oft sogar toxischen anderen Enantiomers geführt. Damit kommt Herstellverfahren zur Darstellung enantiomerenangereicherter asymmetrischer Alkohole als Vorstufen zu diesen Verbindungen besondere Bedeutung zu.

Verfahren zur enzymatischen Racematspaltung von Alkoholen sind bereits bekannt. So wird gemäß Synthesis 1989, 933 - 934 ein racemischer Alkohol in den entsprechenden Buttersäureester überführt und der racemische Ester anschließend in Gegenwart einer Lipase enantioselektiv hydrolysiert. Man erhält ein Enantiomer des Alkohols in freier Form, das andere verbleibt verestert und ist mit konventionellen chemischen Verfahren, etwa Chromatographie abtrennbar und gegebenenfalls durch anschließende chemische Hydrolyse ebenfalls frei erhältlich. Um das gewünschte Enantiomer zu erhalten, sind daher bis zu vier Verfahrensschritte, nämlich chemische Veresterung, enzymatische Hydrolyse, Trennung von Ester und Alkohol und chemische Hydrolyse des Esters erforderlich. Zudem sind Enzyme im allgemeinen in der wässrigen Phase des Reaktionsgemisches der Hydrolyse löslich und daraus nur schwer für eine eventuelle Wiederverwendung gewinnbar. Sie müssen daher an einen wasserunlöslichen Träger fixiert werden.

Alternativ können Enzyme in einem mit Wasser nicht mischbaren organischen Lösungsmittel, in dem sie üblicherweise unlöslich sind, eingesetzt werden. Will man die Notwendigkeit, während der Reaktion Wasser in molaren Mengen einzubringen oder zu entfernen, vermeiden, kann man aber bevorzugt nur solche Prozesse katalysieren, die ohne Bildung oder Verbrauch von Wasser in molaren Mengen ablaufen, also Acylierungen, etwa mit Carbonsäureanhydriden, oder Umesterungen. Gemäß J. Org. Chem. 53 (1988), 5531 läßt sich ein racemischer Alkohol unter Einfluß einer Lipase mittels eines Carbonsäureanhydrids in einem organischen Lösungsmittel enantioselektiv verestern, wodurch beide Enantiomeren voneinander getrennt werden können. Oft wirkt aber die gleichzeitig gebildete Carbonsäure störend auf die Enzymaktivität. In J. Am. Chem. Soc. 107 (1985), 7072 sind als Acylierungsmitel für enzymatische Racematspaltungen von Alkoholen "aktivierte Ester", das sind Ester wenig nucleophiler Alkohole, vorgeschlagen worden. Allerdings können derartige Ester, etwa 2-Haloethylester industriell kaum eingesetzt werden. Des weiteren sind diese Umsetzungen reversibel und damit unvollständig.

In der EP-A-0 321 918 ist ein Verfahren zur enzymatischen Racematspaltung von Alkoholen durch Acylierung mit oder in Vinylacetat oder Vinylchloracetat unter Einfluß einer Lipase beschrieben. Der im Verlauf der Reaktion entstehende Vinylalkohol tautomerisiert zu Acetaldehyd, welcher gasförmig aus dem Reaktionsgemisch entweicht und die Umsetzung irreversibel macht. Nach der enzymatischen Acylierung liegt wiederum ein Enantiomer des Alkohols als Ester, in diesem Fall als Acetat oder als Chloracetat, das andere in freier Form vor. Es hat sich jedoch gezeigt, daß die Abtrennung des gebildeten freien Alkohols von seinem enantiomeren Acetat oder Chloracetat vor allem in größerem Maßstab schwierig ist, da sich die zu trennenden Alkohol-Esterpaare in ihren physikalischen Eigenschaften wie Siedepunkten oder Löslichkeiten oft wenig unterscheiden oder überhaupt schwer trennbare Azeotrope bilden. Dieser schlechten Trennbarkeit wird gemäß der noch nicht veröffentlichten Europäischen Patentanmeldung Nr. 94101158.7 insofern Rechnung getragen, als die Verwendung längerkettiger Vinylester, etwa von Vinyllaurat oder Vinylpalmitat zur enantiomerenreinen Herstellung von Alkinolen empfohlen wird. Dadurch läßt sich der Alkohol zumeist destillativ vom Ester trennen, und das Verfahren wird industriell anwendbar.
Das hohe Molekulargewicht derartiger Vinylester bewirkt allerdings, daß sie besonders bei der Herstellung von Alkoholen mit geringem Molekulargewicht in beträchtlichem gewichtsmäßigen Überschuß eingesetzt werden müssen, sodaß in den entsprechenden Reaktionsgemischen namentlich gegen Ende der Reaktion der nunmehr enantiomerenangereicherte Alkohol nur mehr in sehr geringen Anteilen vorliegt, was einerseits seine Isolierung erschwert, anderseits zu einer schlechten Raum-Zeit-Ausbeute entsprechender Prozesse führt.

Unerwarteterweise wurde nun gefunden, daß diese Nachteile vermieden werden können, wenn zur enzymatischen Racematspaltung asymmetrischer Alkohole nicht die Vinylester von Monocarbonsäuren, sondern die C₂₋ oder C₃₋ Alkenylester von zwei- und mehrbasischen Carbonsäuren eingesetzt werden. Da sich alle Alkenylestereinheiten des Moleküls am enzymatischen Austausch beteiligen, erhält man Ester, die leicht vom nicht umgesetzten Alkohol abgetrennt werden können, da sie große Molekulargewichtsunterschiede zum Alkohol aufweisen.
Überdies ist durch die Anwesenheit mehrerer Alkenylestergruppen in einem Molekül des Acylierungsmittels die Dichte der Alkenylestergruppen sehr groß, sodaß der zu spaltende Alkohol in hoher Konzentration im Reaktionsgemisch vorliegt.

Gegenstand der Erfindung ist daher ein Verfahren zur Racematspaltung asymmetrischer Alkohole der Formel 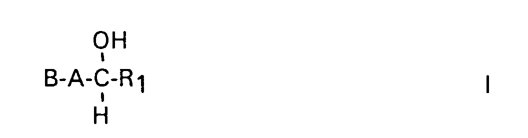 in der
- R₁: COOH, COOC₁-C₄-Alkyl, CN, C₁-C₄-Alkyl, das geradkettig oder verzweigt, gesättigt oder ungesättigt und gegebenenfalls ein- oder mehrfach durch Halogen, C₁-C₄-Alkoxy, Methylendioxy, Aethylendioxy, NH₂, C₁-C₄-Alkylamino, NH-SO₂CH₃, COCH₃, COOH, COOC₁-C₄-Alkyl, NO₂, CN, N₃ substituiert sein kann,
- A: entweder eine Einfachbindung, C₁-C₄-Alkylen oder C₂-C₆-Alkenylen, und
- B: Phenyl, Naphthyl, Pyridyl, Pyrimidyl, Furyl, Thienyl, Imidazolyl, C₁-C₂₀-Alkyl, das geradkettig oder verzweigt, gesättigt oder durch eine oder mehrere Doppel- oder Dreifachbindungen ungesättigt sein kann, wobei eine oder mehrere Methylengruppen durch eine Ketogruppe, durch O, durch NH oder durch N-Alkyl(C₁-C₄) ersetzt sein können, C₃-C₇-Cycloalkyl, das gesättigt oder ungesättigt sein kann, wobei eine Methylengruppe gegebenenfalls durch eine Ketogruppe und eine oder zwei Methylengruppen gegebenenfalls durch O oder NH ersetzt sein können, wobei der Rest B ein- oder mehrfach durch durch Halogen, C₁-C₄-Alkoxy, Methylendioxy, Aethylendioxy, NH₂, C₁-C₄-Alkylamino, NH-SO₂CH₃, CO-CH₃, COOH, COOC₁-C₄-Alkyl, NO₂, CN, N₃ substituiert sein kann, oder
- A: eine Einfachbindung und R₁ und B gemeinsam eine C₃-C₈-Alkylen- oder - Alkenylengruppe, in der 2 Methylengruppen zusätzlich über eine weitere C₁-C₄-Alkylenkette verbrückt sein können, wobei eine oder mehrere Methylengruppen durch eine Ketogruppe, durch O, NH oder N-Alkyl(C₁-C₄) ersetzt sein können, und wobei der Ring aus R₁ und B gegebenenfalls ein- oder mehrfach durch Halogen, C₁-C₄-Alkyl, das geradkettig oder verzweigt sein kann, durch C₁-C₄-Alkoxy, Methylendioxy, Aethylendioxy, NH₂, C₁-C₄-Alkylamino, NH-SO₂CH₃, CO-CH₃, COOH, COOC₁-C₄-Alkyl, NO₂, CN, N₃ substituiert sein kann,
bedeuten, das dadurch gekennzeichnet ist, daß
ein asymmetrischer Alkohol der Formel I mit einem C₂- oder C₃-Alkenylester einer C₂-C₁₀-Alkan- oder -Alken-di- oder -tricarbonsäure in Gegenwart einer Lipase umgesetzt und anschließend der verbleibende nunmehr enantiomerenreine Alkohol der Formel I isoliert und gegebenenfalls das andere Enantiomere aus dem entstandenen Ester gewonnen wird.

Asymmetrische Alkohole der Formel I sind bekannt und/oder durch bekannte Methoden herstellbar. Sie können als racemisches Gemisch oder als Gemisch, in dem bereits eines der beiden Enantiomeren angereichert vorliegt, eingesetzt werden. Es sind dies beispielsweise Betablocker oder Bausteine dafür, wie Phenyl- oder Naphthylalkanolamine, in denen B ein substituierter Phenyl- oder Naphthylring, A eine Einfachbindung und R₁ ein Alkylaminomethylrest ist, oder Zwischenprodukte hiefür, in denen R₁ etwa ein Haloalkylrest ist. Solche Betablocker sind beispielsweise Sotalol oder Nifenalol. Weitere herstellbare Asymmetrische Alkohole sind Alkylalkinylcarbinole (R₁ = Alkyl, B = Alkinyl und A eine Einfachbindung), die etwa Bausteine für Lipoxygenasehemmer darstellen, oder Verbindungen vom Typ 2-Hydroxycarbonsäurenitril (R₁ = CN, B = substituiertes Alkyl), welche wichtige Zwischenprodukte für die Herstellung von Aminosäuren, wie Gammaaminobetahydroxybuttersäure (GABOB) darstellen. Ähnliche Verbindungen, in denen R₁ = CN, A = (CH₂)₂, B = Phenyl ist, sind Zwischenprodukte zur Synthese von ACE-Hemmern wie Enalapril. Viele cyclische und bicyclische Alkohole wie 2-Alkyl-4-hydroxy-cyclohexenone stellen Zwischenprodukte für die Herstellung von Prostaglandinen dar.

C₂- oder C₃-Alkenylester von Alkan- oder Alken-di- oder -tricarbonsäuren sind beispielsweise die Ester der Oxalsäure, Malonsäure, Bernsteinsäure, Glutarsäure, Adipinsäure, Pimelinsäure, Korksäure, Azelainsäure, Sebacinsäure, der Fumar- oder Maleinsäure oder der Aconitsäure. Bevorzugt sind die Ester der Adipinsäure, Bernsteinsäure, Aconitsäure. Bevorzugte Ester sind die Vinylester. Unter C₂- oder C₃-Alkenylestern sind die Vinyl-, Propenyl- oder Isopropenylester zu verstehen.

Unter Lipasen sind zur Racematspaltung geeignete Lipasen zu verstehen, wobei Lipasen aus Schweineleber, Schweinepankreas sowie aus Mikroorganismen wie Candida, Mucor, Rhizopus, Penicillium, Aspergillus, Pseudomonas bevorzugt sind. Besonders bevorzugt sind käufliche Lipasen, ganz bevorzugt Lipasen aus Candida oder Pseudomonas. Die Lipase kann dabei in gereinigter oder teilweise gereinigter Form oder in Form des Mikroorganismus selbst, in freier oder immobilisierter Form eingesetzt werden.

Die Durchführung des erfindungsgemäßen Verfahrens kann nach einer in der EP-A-0 321 918 geoffenbarten Verfahrensweisen erfolgen. Dazu wird eine Lipase und ein Alkohol der Formel I zusammen mit einem C₂- oder C₃-Alkenylester vorgelegt. Pro Mol Alkohol wird dabei mindestens diejenige Menge an Alkenylester eingesetzt, die ein halbes Mol Alkenylestergruppen enthält. Die nötige Menge an Lipase ist von der chemischen Beschaffenheit des Alkohols und des Alkenylesters, von der gewünschten Reaktionszeit und von der Art der Lipase abhängig und kann für jeden Fall durch einen Vorversuch leicht ermittelt werden.

Die Reaktionsmischung wird zusammen mit der Lipase bei Temperaturen von - 10°C bis zur Desaktivierungstemperatur der eingesetzten Lipase, bevorzugt bei der Temperatur, bei der die Lipase ihre höchste Aktivität besitzt und die vom Hersteller im allgemeinen angegeben ist, vorteilhafterweise gerührt oder geschüttelt. Es ist aber auch möglich, die Lipase in einem Modul, beispielsweise in einer Säule, vorzulegen und die Mischung, die den Alkohol und den Alkenylester enthält, im Kreislauf durch dieses Modul zu leiten. Dabei werden die Alkenylestergruppen des Acylierungsmittels sequentiell in Ester eines Enantiomers des asymmetrischen Alkohols überführt, während das andere Enantiomer des asymmetrischen Alkohols im wesentlichen unverändert bleibt. Der freigesetzte Alkenylalkohol tautomerisiert zur entsprechenden Carbonylverbindung, welche nicht mehr am Reaktionsgeschehen teilnimmt.

Das Fortschreiten der Reaktion, das heißt, der sequentielle Austausch der Alkenylestergruppen gegen Estergruppen eines Enantiomers des asymmetrischen Alkohols, wird durch übliche Methoden, beispielsweise durch Gaschromatographie verfolgt. Da eine Lipase zwar bevorzugt eines der beiden Enantiomeren, aber im allgemeinen auch das zweite Enantiomer umsetzen kann, wird in passenden Abständen der Enantiomerenüberschuß ee des nicht umgesetzten Alkohols oder des gebildeten Esters mit mit Hilfe geeigneter Methoden, beispielsweise durch Bestimmung der optischen Drehung oder durch Chromatographie an einer chiralen Phase, gemessen. Nach Erreichen des gewünschten Umsetzungsgrades, der vom gewünschten Produkt und dessen gewünschtem Enantiomerenüberschuß abhängig ist, wird die Reaktion abgebrochen. Zur Aufarbeitung des Reaktionsgemisches wird die Lipase gegebenenfalls beispielsweise durch Abfiltrieren oder Abzentrifugieren aus dem Reaktionsgemisch abgetrennt und der Rückstand einer Trennoperation wie etwa Extraktion, Destillation oder Chromatographie unterworfen. Bevorzugt ist dabei eine Destillation, die gerade durch den erfindungsgemäßen Einsatz von Alkenylestern mehrbasiger Carbonsäuren besonders wirksam ist, da sich die Siedepunkte von Alkohol, dessen Ester, Alkenylester, und der aus dem Alkenylester entstandenen Carbonylverbindung für eine einfache, destillative, hochwirksame Trennung genügend unterscheiden. Wird jenes Enantiomer gewünscht, das das im Verlauf der Reaktion bevorzugt verestert wurde, kann nach Isolierung dieses Esters aus dem Reaktionsgemisch eine Esterhydrolyse, gegebenenfalls ebenfalls in Gegenwart einer Lipase, durchgeführt werden.

Es hat sich gezeigt, daß der Zusatz eines organischen Lösungsmittels zur Reaktionsmischung die Reaktionsgeschwindigkeit positiv beeinflußt. In einer bevorzugten Ausführungsform wird daher der Reaktionsmischung ein organisches Lösungsmittel zugesetzt, wodurch die Viskosität der Reaktionsmischung in vorteilhafter Weise gesenkt wird. Als organische Lösungsmittel eignen sich gegebenenfalls halogenierte aliphatische oder aromatische Kohlenwasserstoffe wie z.B. Pentan, Hexan, Cyclopentan, Toluol, Xylole, Dichlormethan, Dichlorethan, Chlorbenzole, Ether, wie z.B. Diethylether, Tetrahydrofuran, Dioxan, Ester, wie z.B. Essigsäureethyl-, -butylester, oder Mischungen aus solchen Lösungsmitteln, wobei halogenierte aliphatische oder aromatische Kohlenwasserstoffe bevorzugt sind. Das organische Lösungsmittel wird in Mengen von 0,1 bis 70 Vol.%, bevorzugt von etwa 0,5 bis 60 Vol.% bezogen auf die gesamte Reaktionsmischung zugesetzt.

Mit Hilfe des erfindungsgemäßen Verfahrens läßt sich ein Gemisch zweier Enantiomere eines asymmetrischen Alkohols durch enzymkatalysierte stereoselektive Acylierung in guter Raum-Zeit- Ausbeute in ein technisch leicht trennbares Gemisch, das das eine Enantiomer des asymmetrischen Alkohols in unveränderter Form, das andere Enantiomer in Form eines Esters enthält, überführen. Das Verfahren liefert enantiomerenreine Alkohole oder Ester, wobei je nach Umsetzungsgrad ein Enantiomerenüberschuß von über 90 % im unveränderten Alkohol oder im Ester erhalten wird. Das Verfahren stellt daher eine Bereicherung der Technik dar.

### Beispiele

### Beispiele 1-4:

Zu 7,13 mmol eines racemischen Alkohols wurden 0,92 g (4,63 mmol) Divinyladipat, 0,02 ml Natriumphosphatpuffer (0,1 M, pH 7,0), 4,5 ml o-Xylol sowie 0,3 g Pseudomonas-Lipase (Amano PS) zugegeben. Das Reaktionsgemisch wurde bei 40°C geschüttelt. Der Enantiomerenüberschuß des nicht umgesetzten Alkohols wurde als Trifluoracetylderivat mittels GC an einer Cyclodextrinsäule bestimmt. Die erzielten Ergebnisse sind in Tabelle I aufgelistet.

**Tabelle I**

| Beispiel Nr. | Alkohol | Reaktionszeit | % ee |
|---|---|---|---|
| **1.** | **3-Methyl-2-butanol** | 168 Std. | **94** |
| **2.** | **1-Octin-3-ol** | 168 Std. | **95** |
| **3.** | **2-Octanol** | 23 Std. | **98** |
| **4.** | **2-Chlor-1-Phenylethanol** | 188 Std. | **95** |

### Beispiel 5

Zu 50 g (0,713 mol) R,S-But-3-in-2-ol wurden 87,17 g (0,44 mol, 0,62 Äquivalente bezogen auf den Alkohol) Divinyladipat, 1,50 ml Natriumphosphatpuffer (0,1 M, pH 7,0), 450 ml o-Xylol sowie 30 g Pseudomonas-Lipase (Amano PS) zugegeben. Das Reaktionsgemisch wurde bei 40°C gerührt. Der Enantiomerenüberschuß ee des nicht veresterten Alkohols wurde mittels GC an einer Cyclodextrinsäule bestimmt. Nach Erreichen von 95% ee wurde die Reaktion durch Abfiltrieren des Enzyms abgebrochen und das Filtrat zur Isolation von S-But-3-in-2-ol fraktioniert destilliert, wobei 12 g S-But-3-in-2-ol mit 95 % ee erhalten wurden.

### Beispiel 6

Zu 1,0 g (0,014 mol) R,S-But-3-in-2-ol wurden 1,84 g (0,009 mol, 0,65 Äquivalente bezogen auf den Alkohol) Divinyladipat, 30 µl Natriumphosphatpuffer (0,1 M, pH 7,0), 9 ml o-Xylol sowie 0,6 g Pseudomonas-Lipase (Amano PS) zugegeben. Das Reaktionsgemisch wurde bei 40°C gerührt. Die Reaktion wurde mittels Gaschromatographie an einer Cyclodextrinsäule verfolgt, und dabei festgestellt, daß beide Vinylestergruppen reagieren. Zunächst entsteht But-3-in-2-yl-vinyl-adipat, welches zu Di-but-3-in-2-yl-adipat weiterreagiert.

## Patentansprüche

1. Verfahren zur Racematspaltung asymmetrischer Alkohole der Formel 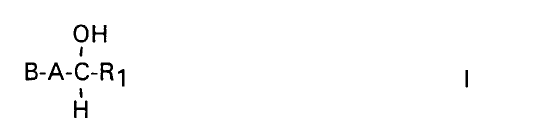 in der
R₁ COOH, COOC₁-C₄-Alkyl, CN, C₁-C₄-Alkyl, das geradkettig oder verzweigt, gesättigt oder ungesättigt und gegebenenfalls ein- oder mehrfach durch Halogen, C₁-C₄-Alkoxy, Methylendioxy, Aethylendioxy, NH₂, C₁-C₄-Alkylamino, NH-SO₂CH₃, COCH₃, COOH, COOC₁-C₄-Alkyl, NO₂, CN, N₃ substituiert sein kann,
A entweder eine Einfachbindung, C₁-C₄-Alkylen oder C₂-C₆-Alkenylen, und
B Phenyl, Naphthyl, Pyridyl, Pyrimidyl, Furyl, Thienyl, Imidazolyl, C₁-C₂₀-Alkyl, das geradkettig oder verzweigt, gesättigt oder durch eine oder mehrere Doppel- oder Dreifachbindungen ungesättigt sein kann, wobei eine oder mehrere Methylengruppen durch eine Ketogruppe, durch O, durch NH oder durch N-Alkyl(C₁-C₄) ersetzt sein können, C₃-C₇-Cycloalkyl, das gesättigt oder ungesättigt sein kann, wobei eine Methylengruppe gegebenenfalls durch eine Ketogruppe und eine oder zwei Methylengruppen gegebenenfalls durch O oder NH ersetzt sein können, wobei der Rest B ein- oder mehrfach durch durch Halogen, C₁-C₄-Alkoxy, Methylendioxy, Aethylendioxy, NH₂, C₁-C₄-Alkylamino, NH-SO₂CH₃, CO-CH₃, COOH, COOC₁-C₄-Alkyl, NO₂, CN, N₃ substituiert sein kann, oder
A eine Einfachbindung und R₁ und B gemeinsam eine C₃-C₈-Alkylen- oder - Alkenylengruppe, in der 2 Methylengruppen zusätzlich über eine weitere C₁-C₄-Alkylenkette verbrückt sein können, wobei eine oder mehrere Methylengruppen durch eine Ketogruppe, durch O, NH oder N-Alkyl(C₁-C₄) ersetzt sein können, und wobei der Ring aus R₁ und B gegebenenfalls ein- oder mehrfach durch Halogen, C₁-C₄-Alkyl, das geradkettig oder verzweigt sein kann, durch C₁-C₄-Alkoxy, Methylendioxy, Aethylendioxy, NH₂, C₁-C₄-Alkylamino, NH-SO₂CH₃, CO-CH₃, COOH, COOC₁-C₄-Alkyl, NO₂, CN, N₃ substituiert sein kann,
bedeuten, dadurch gekennzeichnet, daß
ein asymmetrischer Alkohol der Formel I mit einem C₂- oder C₃-Alkenylester einer C₂-C₁₀-Alkan- oder -Alken-di- oder -tricarbonsäure in Gegenwart einer Lipase umgesetzt und anschließend der verbleibende nunmehr enantiomerenreine Alkohol der Formel I isoliert und gegebenenfalls das andere Enantiomere aus dem entstandenen Ester gewonnen wird.

2. Verfahren gemäß Anspruch 1, dadurch gekennzeichnet, daß
R₁ COOH, CN, C₁-C₄-Alkyl, das geradkettig oder verzweigt, gesättigt oder ungesättigt und gegebenenfalls ein- oder mehrfach durch Halogen, C₁-C₄-Alkylamino, COOH, CN, N₃ substituiert ist, bedeutet.

3. Verfahren gemäß Anspruch 1, dadurch gekennzeichnet, daß
A eine Einfachbindung ist.

4. Verfahren gemäß Anspruch 1, dadurch gekennzeichnet, daß
B Phenyl, Furyl, Imidazolyl, C₁-C₂₀-Alkyl, das geradkettig oder verzweigt, gesättigt oder durch eine oder mehrere Doppel- oder Dreifachbindungen ungesättigt sein kann, wobei eine oder mehrere Methylengruppen durch eine Ketogruppe, oder durch O ersetzt sein können, C₃-C₇-Cycloalkyl, das gesättigt oder ungesättigt sein kann, wobei eine Methylengruppe gegebenenfalls durch eine Ketogruppe und eine oder zwei Methylengruppen gegebenenfalls durch O ersetzt sein können, wobei der Rest B ein- oder mehrfach durch durch Halogen, C₁-C₄-Alkoxy, C₁-C₄-Alkylamino, CN, N₃ substituiert sein kann, bedeutet.

5. Verfahren gemäß Anspruch 1, dadurch gekennzeichnet, daß
A eine Einfachbindung und R₁ und B gemeinsam eine C₃-C₈-Alkylengruppe, in der 2 Methylengruppen zusätzlich über eine weitere C₁-C₄-Alkylengruppe verbrückt sein können, wobei eine oder mehrere Methylengruppen durch eine Ketogruppe oder O ersetzt sein können, und wobei der Ring aus R₁ und B gegebenenfalls ein- oder mehrfach durch Halogen, C₁-C₄-Alkyl, das geradkettig oder verzweigt sein kann, durch C₁-C₄-Alkoxy, C₁-C₄-Alkylamino, CN, N₃ substituiert sein kann, bedeutet.

## Claims

1. Process for the resolution of asymmetric alcohols of the formula 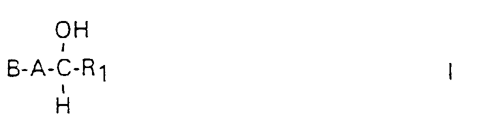 in which
R₁ is COOH, COOC₁-C₄-alkyl, CN, C₁-C₄-alkyl which can be straight-chain or branched, saturated or unsaturated and optionally mono- or polysubstituted by halogen, C₁-C₄-alkoxy, methylenedioxy, ethylenedioxy, NH₂, C₁-C₄-alkylamino, NH-SO₂CH₃, COCH₃, COOH, COOC₁-C₄-alkyl, NO₂, CN, N₃,
A is either a single bond, C₁-C₄-alkylene or C₂-C₆-alkenylene, and
B is phenyl, naphthyl, pyridyl, pyrimidyl, furyl, thienyl, imidazolyl,
C₁-C₂₀-alkyl which can be straight-chain or branched, and saturated or unsaturated by one or more double or triple bonds, where one or more methylene groups can be replaced by a keto group, by O, by NH or by N-alkyl(C₁-C₄),
C₃-C₇-cycloalkyl which can be saturated or unsaturated, where a methylene group can optionally be replaced by a keto group and one or two methylene groups can optionally be replaced by O or NH, where the radical B can be mono- or polysubstituted by by halogen, C₁-C₄-alkoxy, methylenedioxy, ethylenedioxy, NH₂, C₁-C₄-alkylamino, NH-SO₂CH₃, CO-CH₃, COOH, COOC₁-C₄-alkyl, NO₂, CN, N₃, or
A is a single bond and R₁ and B together are a C₃-C₈-alkylene or -alkenylene group, in which 2 methylene groups can additionally be bridged by means of a further C₁-C₄-alkylene chain, where one or more methylene groups can be replaced by a keto group, or by O, NH or N-alkyl(C₁-C₄) and where the ring formed from R₁ and B can optionally be mono- or polysubstituted by halogen, C₁-C₄-alkyl which can be straight-chain or branched, by C₁-C₄-alkoxy, methylenedioxy, ethylenedioxy, NH₂, C₁-C₄-alkylamino, NH-SO₂CH₃, CO-CH₃, COOH, COOC₁-C₄-alkyl, NO₂, CN, N₃,
which is characterized in that
an asymmetric alcohol of the formula I is reacted with a C₂- or C₃-alkenyl ester of a C₂-C₁₀-alkane- or -alkene-di- or -tricarboxylic acid in the presence of a lipase and the remaining alcohol of the formula I which is now enantiomerically pure is then isolated and if desired the other enantiomer is recovered from the ester formed.

2. Process according to Claim 1, characterized in that
R₁ is COOH, CN, C₁-C₄-alkyl which is straight-chain or branched, saturated or unsaturated and is optionally mono- or polysubstituted by halogen, C₁-C₄-alkylamino, COOH, CN or N₃.

3. Process according to Claim 1, characterized in that
A is a single bond.

4. Process according to Claim 1, characterized in that
B is phenyl, furyl, imidazolyl, C₁-C₂₀-alkyl which can be straight-chain or branched, saturated or unsaturated by one or more double or triple bonds, where one or more methylene groups can be replaced by a keto group or by O, C₃-C₇-cycloalkyl which can be saturated or unsaturated, where a methylene group can optionally be replaced by a keto group and one or two methylene groups can optionally be replaced by 0, where the radical B can be mono- or polysubstituted by halogen, C₁-C₄-alkoxy, C₁-C₄-alkylamino, CN or N₃.

5. Process according to Claim 1, characterized in that
A is a single bond and R₁ and B together are a C₃-C₈-alkylene group in which 2 methylene groups can additionally be bridged by means of a further C₁-C₄-alkylene group, where one or more methylene groups can be replaced by a keto group or O and where the ring formed from R₁ and B can optionally be mono- or polysubstituted by halogen, C₁-C₄-alkyl which can be straight-chain or branched, by C₁-C₄-alkoxy, C₁-C₄-alkylamino, CN or N₃.

## Revendications

1. Procédé pour la séparation de racémates d'alcools asymétriques répondant à la formule 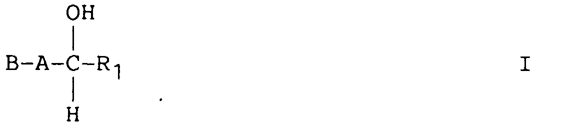 où
R¹ signifie COOH, COO-alkyle en C₁-C₄, CN, un groupe alkyle en C₁-C₄, qui peut être linéaire ou ramifié, saturé ou insaturé et éventuellement mono- ou polysubstitué par un halogène, ou par des groupes alcoxy en C₁-C₄, méthylènedioxy, éthylènedioxy, NH₂, alkylamino en C₁-C₄, NH-SO₂CH₃, COCH₃, COOH, COO-alkyle en C₁-C₄, NO₂, CN ou N₃,
A signifie une liaison simple, un groupe alkylène en C₁-C₄ ou un groupe alcénylène en C₂-C₆, et
B signifie un groupe phényle, naphtyle, pyridyle, pyrimidyle, furyle, thiényle, imidazolyle, alkyle en C₁-C₂₀ qui peut être linéaire ou ramifié, saturé ou insaturé par une ou plusieurs liaisons doubles ou triples, un ou plusieurs groupes méthylène pouvant être remplacés par un groupe céto, par O, par NH ou par un groupe N-alkyle en C₁-C₄, cycloalkyle en C₃-C₇ qui peut être saturé ou insaturé, un groupe méthylène pouvant être remplacé éventuellement par un groupe céto, et un ou plusieurs groupes méthylène pouvant être remplacés éventuellement par O ou NH, le reste B pouvant être mono- ou polysubstitué par un halogène ou par un groupe alcoxy en C₁-C₄, méthylènedioxy, éthylènedioxy, NH₂, alkylamino en C₁-C₄, NH-SO₂CH₃, CO-CH₃, COOH, COO-alkyle en C₁-C₄, NO₂, CN ou N₃, ou
A signifie une liaison simple, et R₁ et B pris ensemble signifient un groupe alkylène ou alcénylène en C₃-C₈, où 2 groupes méthylène peuvent être pontés en outre par une chaîne supplémentaire alkylène en C₁-C₄, un ou plusieurs groupes méthylène pouvant être remplacés par un groupe céto, par O, NH ou un groupe N-alkyle en C₁-C₄, et le noyau formé de R₁ et B pouvant éventuellement être mono- ou polysubstitué par un halogène, un groupe alkyle en C₁-C₄ qui peut être linéaire ou ramifié, par un groupe alcoxy en C₁-C₄, méthylènedioxy, éthylènedioxy, NH₂, alkylamino en C₁-C₄, NH-SO₂CH₃, CO-CH₃, COOH, COO-alkyle en C₁-C₄, NO₂, CN ou N₃,
caractérisé en ce qu'on fait réagir un alcool asymétrique de formule I avec un ester d'alcényle en C₂ ou C₃ d'un acide alcane- ou alcène(en C₂-C₁₀)-di- ou tricarboxylique, en présence d'une lipase, et ensuite, on isole l'alcool de formule I restant, qui présente maintenant des énantiomères purs, et éventuellement, on récupère l'autre énantiomère à partir de l'ester formé.

2. Procédé selon la revendication 1, caractérisé en ce que R₁ signifie COOH, CN, alkyle en C₁-C₄ qui peut être linéaire ou ramifié, saturé ou insaturé et éventuellement mono- ou polysubstitué par un halogène, un groupe alkylamino en C₁-C₄, COOH, CN ou N₃.

3. Procédé selon la revendication 1, caractérisé en ce que A est une liaison simple.

4. Procédé selon la revendication 1, caractérisé en ce que B signifie un groupe phényle, furyle, imidazolyle, alkyle en C₁-C₂₀ qui peut être linéaire ou ramifié, saturé ou insaturé par une ou plusieurs liaisons doubles ou triples, un ou plusieurs groupes méthylène pouvant être remplacés par un groupe céto ou par O, un groupe cycloalkyle en C₃-C₇ qui peut être saturé ou insaturé, un groupe méthylène pouvant être remplacé éventuellement par un groupe céto, et un ou plusieurs groupes méthylène pouvant être remplacés éventuellement par O, le reste B pouvant être mono- ou polysubstitué par un halogène ou par un groupe alcoxy en C₁-C₄, alkylamino en C₁-C₄, CN ou N₃.

5. Procédé selon la revendication 1, caractérisé en ce que A signifie une liaison simple, et R₁ et B pris ensemble signifient un groupe alkylène en C₃-C₈, dans lequel 2 groupes méthylène sont pontés en outre par une liaison supplémentaire alkylène en C₁-C₄, un ou plusieurs groupes méthylène pouvant être remplacés par un groupe céto ou par O, et le noyau formé de R₁ et B pouvant éventuellement être substitué par un halogène, par un groupe alkyle en C₁-C₄ qui peut être linéaire ou ramifié, par un groupe alcoxy en C₁-C₄, alkylamino en C₁-C₄, CN ou N₃.
